# EUROPEAN PATENT SPECIFICATION

(11) **EP 3 082 796 B1**
(45) Date of publication and mention of the grant of the patent: **20.05.2020**
(21) Application number: 14800163.9
(22) Date of filing: 07.11.2014
(51) Int. Cl.: A61K 31/202, A61K 31/685, A23L 33/10, A23L 33/00, A61K 45/06, A61P 43/00

(54) **EFFICACY OF DIETARY DHA-PHOSPHOLIPID FOR BRAIN DHA AND DPA ACCRETION IN NEONATES**
WIRKSAMKEIT VON DIÄTETISCHEM DHA-PHOSPHOLIPID ZUR DHA UND DPA ZUNAHME IM GEHIRN VON NEUGEBORENEN
EFFICACY DU DHA-PHOSPHOLIPID DIETETIQUE POUR L'AUGMENTATION DU DHA ET DU DPA DANS LE CERVEAUX DES NOUVEAUX-NÉS

(30) Priority: 08.11.2013 WO PCT/NL2013/050801
(43) Date of publication of application: 26.10.2016
(73) Proprietor: N.V. Nutricia, 2712 HM Zoetermeer (NL)
(72) Inventor: BARTKE, Nana, NL-3584 CT Utrecht (NL); WOBBES, Barry, 3584 CT Utrecht (NL)
(74) Representative: Nederlandsch Octrooibureau
(86) International application number: PCT/NL2014/050769
(87) International publication number: WO 2015/069108

(56) References cited:
- EP-A1- 2 110 027
- WO-A1-2010/110649
- FR-A1- 2 749 133
- US-A1- 2008 003 330
- ALESSANDRI ET AL: "Docosahexaenoic acid concentrations in retinal phospholipids of piglets fed an infant formula enriched with long-chain polyunsaturated fatty acids: effects of egg phospholipids and fish oils with different ratios of eicosapentaenoic acid to docosahexaenoic acid.", THE AMERICAN JOURNAL OF CLINICAL NUTRITION, vol. 67, no. 3, 1 March 1998 (1998-03-01), pages 377-385, XP055098740, US ISSN: 0002-9165
- SHYH-HWA LIU ET AL: "Docosahexaenoic acid and phosphatidylserine supplementations improve antioxidant activities and cognitive functions of the developing brain on pentylenetetrazol-induced seizure model", BRAIN RESEARCH, ELSEVIER, AMSTERDAM, NL, vol. 1451, 24 February 2012 (2012-02-24), pages 19-26, XP028412001, ISSN: 0006-8993, DOI: 10.1016/J.BRAINRES.2012.02.060 [retrieved on 2012-03-03]

## Description

### Field of the Invention

The present invention relates to the use of DHA carried by a phospholipid for increasing the amount of DPA or DHA and DPA in tissues and/or organs of a young mammal. In particular, the present invention relates to the use of an infant formula comprising DHA carried by a phospholipid for increasing the amount of DPA or DHA and DPA in tissues and/or organs of a young mammal.

### Background to the invention

Mother's milk is known as the "Golden Standard" when it comes to providing nutrition to infants. However, for a variety of reasons it may not be possible for a mother to sufficiently breast feed her infant to provide the daily required amounts of nutrients. Infant formulae have been developed to serve as a substitute in situations where breast feeding is inadequate or in cases this cannot be practiced at all.

One of the ingredients or components typically comprised by present day infant formulae are long chain polyunsaturated fatty acids (LC-PUFAs). The reported health benefits from LC-PUFAs for infants are numerous. For instance, as the developing brain accumulates large amounts of DHA during the pre- and postnatal development which continues throughout the first 2 years after birth, intake of LC-PUFAs, in particular DHA, is important to support the normal physical development of the brain, eyes and nerves and in particular in children in that age category. Also, DHA has a structural and functional role in the retina and its intake contributes to the visual development of infants up to 12 months of age. Inclusion of LC-PUFAs in infant formulae is thus desired and often advocated in nutritional regulatory bodies (cf. EU Commission Directive 2006/141/EC).

The natural lipid classes that carry most LC-PUFAs in foods are triacylglycerols ['TAG'] and phospholipids ['PL']. In mature human milk, approximately 85% of LC-PUFAs are in the form of triacylglycerols, and 15% of LC-PUFAs are present as phospholipids; the majority of DHA in absolute numbers is found in triacylglycerols because of the overwhelming prevalence of triacylglycerols versus phospholipids in the milk.

After ingestion, both PL-bound LC-PUFAs and TAG-bound LC-PUFAs are digested, absorbed, transported to their target organs and taken up by the various tissues. However, the *in vivo* metabolism of LC-PUFAs, in particular the specific interactions of desaturation and elongation enzymes part of the biosynthetic pathways involving DHA, EPA and DPA molecules and their inter-molecular conversion in mammalian tissues, are not fully elucidated yet. This makes that the full value of these nutritional compounds comprised by infant formulae may at present not be fully appreciated and/or even underestimated despite the presently available knowledge. Furthermore, present day legislation typically prohibits the inclusion of phospholipids above a certain threshold value. Focus has largely been on DHA, EPA, and AA since those LC-PUFAs have been recognized as having important roles in sustaining infant growth and development. On top of this, similar LC-PUFAs such as docosapentaenoic acid (DPA) in purified form suited for infant nutrition are expensive ingredients. In light of these constraints, having knowledge on the metabolic fate, and tissue and organ distribution of LC-PUFAs comprised by phospholipids can be of valuable use for further optimizing infant formulae with respect to individual LC-PUFA contents and/or advocating its use and benefits.

WO 96/10922 discloses a fat mixture, and a food containing said fat mixture, that comprises arachidonic acid and docosahexaenoic acid which are present as phospholipids in a certain proportion. In particular a fat mixture is disclosed based on animal and plant, including optionally microbial, oils and/or fats and lecithins containing long-chain polyunsaturated fatty acids, characterized in that the arachidonic acid present in the fat mixture in the form of phospholipids makes up 0.2 to 3.0 mg/g total fat and the docosahexaenoic acid present in the form of phospholipids makes up 0.1 to 2.0 mg/g total fat, and that the arachidonic acid and docosahexaenoic acid present in the fat mixture in the form of triglycerides each make up 0.05 to 1.5 wt %, based on the sum of the fatty acids present in the form of triglycerides.

Graf et al (Prostaglandins, Leukotrienes and Essential Fatty Acids 83 (2010) 89-96) have shown that orally dosed DHA reached the brain in 2-, 4- and 10-week-old rats. Also, in 10-week-old rats, tissues and organs such as liver, brain, kidney and anterior uveal tract (retina) accumulated 2-3 times more ¹⁴C-DHA derived radioactivity after ¹⁴C-DHA-PC dosing compared with ¹⁴C-DHA-TAG dosing.

EP 2 110 027 A1 by Nestec S.A. (WO 2009/121839 A1) discloses a maternal food that can be administered to mothers during pregnancy and lactation and that increases the DHA levels in the neonates, for example to support brain and retina development of the neonate. Said maternal food composition was not only effective when administered directly to the infant, but was also effective when administered to the mother. Said source of lipids includes at least one LC-PUFA selected from the group consisting of arachidonic acid, eicosatrienoic acid, eicosapentaenoic acid and docosapentaenoic, docosahexaenoic acid (DHA) which can be in the form selected from the group consisting of phospholipids, phosphotidylcholine, phosphatidylethanolamine, N-Acylphosphatidylethanolamine, phosphatidylinositol and phosphatidylserine.

Hitherto, while human milk is known to comprise DPA, the focus in the art has been on increasing DHA brain levels.

### Summary of the invention

The present invention relates to a composition for use in increasing the amount of DPA or the amount of DHA and DPA in tissues and/or organs of a human infant at risk of or suffering from impaired DPA levels in tissue and/or organs, wherein said composition comprises a DHA comprising phospholipid (PL-DHA) and comprises at least 0.1 wt% omega-3 PUFAs, based on total fat. The present invention also relates to a composition for use in increasing the amount of DPA or the amount of DHA and DPA in tissues and/or organs of a human infant suffering from impaired DPA levels in tissue and/or organs, wherein said composition comprises a DHA comprising phospholipid (PL-DHA).

In particular, the present invention relates to achieving an increase in the amount of DPA or the amounts of DHA and DPA in the brain or more in particular in grey matter of the brain of the human infant. More in particular, the increase in DPA or the amounts of DHA and DPA is achieved in tissues and/or organs that comprise or consist of membrane-rich tissue, neural tissue, brain tissue, cerebral cortex grey matter, brain grey matter, synaptosomes, retina, the liver, the brain and/or the eyes of the young mammal, preferably the brain, retina and liver, more preferably the brain. The mammal is a human infant.

DPA is an expensive LC-PUFA which is less investigated than other LC-PUFA's such as EPA, DHA, ARA or ALA. Still there is a growing body of research revealing that DPA is an important component responsible for inflammation, cognition and heart effects within the mammal's body. With the present knowledge in mind that upon consumption of PL-DHA, this compound can be found as DPA in certain organs and tissue in infants, it has now thus become possible to make a more conscious choice of not including DPA, or to diminish DPA levels in infant formulae, knowing that DPA levels in the infant can be targeted by administering PL-DHA. Without the need for expensive DPA ingredients, it is still possible to increase DPA yields in the target organs and tissues in the growing infant. This even holds in situation where no DPA is included in the feed regime, provided that PL-DHA is present in the formula. Opting to include PL-DHA in infant formula instead of DPA allows one to produce a less costly infant formula.

Throughout the application, 'DHA comprising phospholipid' and PL-DHA will be used interchangeably and means that one or at least one DHA chain is chemically linked to or esterified with a phospholipid chain, i.e. the dietary composition comprises phosholipids which comprise DHA. In the dietary compositions of the invention at least a part of the phospholipids present in the composition thus comprise DHA. In a preferred embodiment, the dietary DHA is present in the sn-2 position of the phospholipid carrier. In the context of the invention, the terminology 'dietary DHA comprising phospholipid' clarifies that PL-DHA is part of the diet or supplemented to the diet of the young mammal, thus distinguishing from the increased DPA or DHA and DPA levels in the mammal's tissues and organs strived for.

In a preferred embodiment, the phospholipid present in the dietary composition is an egg phospholipid. More preferably, the phospholipid is selected from the group consisting of phosphatidylcho line, phosphatidylserine, phosphatidylethanolamine, lysophosphatidylcholine, sphingomyelin, lysophosphatidylethanolamine and phosphatidylinositol. Preferably the phospholipid comprises at least phosphatidylcholine or the phospholipid comprises a mixture of egg phospholipids comprising phosphatidylcholine in an amount of between 60 and 80 wt% of the phospholipid mixture. More preferably, the phospholipid is part of a mixture wherein phosphatidylcholine is present in an amount of between 65 and 75 wt% of the phospholipid mixture, most preferably between 70 and 75 wt%. Other preferred phospholipids include krill oil phospholipids, fish oil phospholipids and phospholipids derived from algae.

In light of the reported health benefits for infants, the dietary DHA comprising phospholipid is present in an infant formula in a preferred embodiment. Preferably, the human infant has an age of between 0 and 36 months, preferably between 0 and 24 months, more preferably between 0 and 12 months. Consequently, in a preferred embodiment, the dietary DHA comprising phospholipid is comprised by an infant formula which formula is adapted to feed infants of age of between 0 and 36 months, preferably between 0 and 24 months, more preferably between 0 and 12 months or 0 to 6 months. In another preferred embodiment, the infant formula is designed for preterm infants.

In a preferred embodiment, the amount of DHA shows at least a 1.5-fold increase, more preferably an about 2-fold increase, and the amount of DPA shows at least a 2-fold increase, preferably a 2- to 4-fold increase, preferably a 2- to 3-fold, more preferably a 2.8 fold increase.

In a preferred embodiment, the amount of DPA shows at least a 2-fold increase, preferably a 2- to 4-fold increase, preferably a 2- to 3-fold, more preferably a 2.8 fold increase, in the brain of the young mammal.

In a preferred embodiment, the amount of DPA shows at least a 2-fold increase, preferably a 2- to 4-fold increase, preferably a 2- to 3-fold, more preferably a 2.6 fold increase, in the liver of the young mammal.

In a preferred embodiment, the amount of DPA shows at least a 2-fold increase, preferably a 2- to 4-fold increase, preferably a 2- to 3-fold, more preferably a 2.4 fold increase, in the retina of the young mammal.

In a preferred embodiment, the amount of DPA shows at least a 2-fold increase, preferably a 2- to 4-fold increase, preferably a 2- to 3-fold, more preferably a 2.8 fold increase, in the synaptosomes of the young mammal.

In a preferred embodiment, the increase in the amount of DPA or the amounts of DHA and DPA is achieved by a conversion from PL-DHA. More preferably, the increase in the amount of DPA occurs simultaneously and/or concomitantly with and/or as a result of the DHA increase.

The invention also pertains to a composition for use in increasing the amount of DPA or the amount of DHA and DPA in tissues and/or organs of a human infant suffering from impaired DPA levels in tissue and/or organs, wherein said composition comprises a DHA comprising phospholipid (PL-DHA). Preferably, said human infant is a preterm infant. Disclosed is a method of increasing the amount of DPA or the amount of DHA and DPA in tissues and/or organs of a young mammal, preferably a human infant, by allowing the young mammal to consume a dietary DHA comprising phospholipid (PL-DHA). Preferably, said human infant is a preterm infant. In one aspect, the invention pertains to a non-therapeutic method for supporting healthy brain development of a young mammal, said method involving increasing the amount of DPA or the amount of DHA and DPA in tissues and/or organs of a young mammal, by allowing the young mammal to consume a dietary DHA comprising phospholipid (PL-DHA), wherein the composition comprises at least 0.1 wt% omega-3 PUFAs, based on total fat, said young mammal preferably being a human infant.

### Detailed description of the invention

The present invention relates to a composition for use in increasing the amount of DPA or the amount of DHA and DPA in tissues and/or organs of a human infant at risk of or suffering from impaired DPA levels in tissue and/or organs, wherein said composition comprises a DHA comprising phospholipid (PL-DHA) and comprises at least 0.1 wt% omega-3 PUFAs, based on total fat. In particular, the present invention relates to achieving an increase in the amount of DPA or the amounts of DHA and DPA in the grey matter of the brain of the young mammal. More in particular, the increase in DPA or DHA and DPA is achieved in tissues and/or organs that comprise or consist of neural tissue, brain tissue, cerebral cortex grey matter, brain grey matter, retina, the liver, the brain and/or the eyes of the human infant. The inventors of the present invention have surprisingly found that supplementation of food with dietary DHA comprising phospholipids results in an increase in the amounts of DHA in said biological target tissues and organs which is accompanied by an increase in DPA levels.

LC-PUFAs are important ingredients for infant nutrition. LC-PUFAs play an important role during the rapid development of the infant brain of mammals and human infants. Large amounts of DHA are deposited in the developing brain and retina during prenatal growth and early postnatal growth during which accretion of DHA in brain tissue continues at high levels until approximately 2 years of age. DHA is the most abundant omega-3 fatty acid in the mammalian brain, accounting for 8-14% of fatty acids at the perinatal period in primates including humans. In humans, DHA accumulates at an accelerating rate from the middle of gestation reaching an inflection point in the first months after birth and continuing well past the plateau of brain weight to plateau at about 18 years of age and remain stable through the end of life. After birth, LC-PUFAs are selectively incorporated, retained, and highly concentrated in the phospholipids bilayer of biologically active brain and retinal neural membranes. DHA comprises about 40% of the total fatty acids of the retinal photoreceptor membranes making it a major membrane constituent. Furthermore, LC-PUFAs, including DHA, affect membrane function, photoreceptor differentiation, activation of the visual pigment rhodopsin, the activity of several enzymes, the function of ion channels, and the levels and metabolism of neurotransmitters and eicosanoids. In preterm infants the supply of LCPUFAs is prematurely interrupted, and hence preterm infants are at higher risk for inadequate DHA accumulation due to the interruption of the placental supply. Thus LC-PUFAs, in particular DHA, support healthy brain development in young children along with supporting retinal development and immune function.

DPA, another omega-3 LC-PUFA species (typically denoted as 22:5(n-3)), has been less extensively studied than DHA and EPA due to its naturally low levels in food additives such as egg yolk and krill oil and more limited availability in pure form. It has been implicated though in inhibition of aggregation in platelets and there is evidence that DPA is important in wound-healing processes, endothelial cell migration ability and development of thrombosis. Furthermore, DPA has transcriptional activity and reduces the expression of lipogenic genes, hepatic enzyme activity of FAS and malic enzyme. As well, DPA has a positive role in reducing the expression of inflammatory genes and has been implicated in lowering cholesterol activity (in particular reducing plasma total cholesterol and non-high-density-lipoprotein cholesterol) and to significantly improve aorta function (in particular to lower aorta tension and to improve aorta relaxation). However, to date, specific inclusion of DPA as a source of LC-PUFAs in infant formulae is not advocated in regulatory bodies. It is further disclosed that the dietary DHA comprising phospholipid provides for improving the health of a young mammal, preferably a human infant, through increasing the amounts of DPA or of both DHA and DPA in tissues and/or organs of said mammal. In particular, the improving of the health comprises reducing of inflammation, improving immunological responses, reducing risk of thrombosis, improving wound-healing processes and/or lowering cholesterol activity (in particular reducing plasma total cholesterol and non-high-density-lipoprotein cholesterol) and to significantly improve aorta function (in particular to lower aorta tension and to improve aorta relaxation).

The herein presented data that phospholipid comprised dietary DHA is converted into DPA in the various tissues and organs of infants or immature mammals as mentioned herein can be used to provide a more complete picture of the beneficial effects of phospholipid comprised dietary DHA. Also, the insight that DHA is converted into DPA in neonatal mammals provides an advantage in that this compound need not be included specifically into infant formulae to attain the known health effects since more knowledge is now at hand that DPA levels will increase through the supplementation of DHA.

In a preferred embodiment, the phospholipid which comprises the dietary DHA is an egg phospholipid. More preferably, the phospholipid is selected from the group consisting of phosphatidylcho line, phosphatidylserine, phosphatidylethanolamine, lysophosphatidylcholine, sphingomyelin, lysophosphatidylethanolamine and phosphatidylinositol, preferably phosphatidylcholine or any mixture of said egg phospholipids but at least comprising phosphatidylcholine. The dietary DHA comprising phospholipid according to the invention is suitable for use in infant formula. An advantage of using egg phospholipids are their superior digestibility due to natural emulsification properties inherent of egg phospholipids. Other preferred phospholipids include those obtainable from or present in krill oil, fish oil, and/or isolated or obtainable from algae.

In a preferred embodiment, the dietary DHA is comprised by a mixture of two or more, three or more, four or more, or five or more different phospholipids. It is preferred that the phospholipid mixture comprises at least 60 wt%, more preferably at least 65 wt%, most preferably at least 70 wt% phosphatidylcholine, based on the total weight of the phospholipids. Preferably, said mixture of phospholipids (comprising DHA) comprises at least 70-75 wt% phosphatidylcholine. It may further comprise the following additional phospholipids in the indicated amounts: 15-20 wt% phosphatidylethanolamine; 3-4 wt% lysophosphatidylcholine; 2-3wt% sphingomyelin; 1-2 wt% lysophosphatidylethanolamine, and 2-3 wt% phosphatidylinositol. In an alternative embodiment, said mixture of phospholipids comprises at least 70-75 wt% phosphatidylcholine and further comprises the following additional phospholipids in the indicated amounts: 20-25 wt% phosphatidylethanolamine, 0.5-2 wt% phosphatidylinositol and 2-8 wt% cardiolipin.

In a preferred embodiment, the dietary DHA is comprised by a phospholipid, meaning it is bound to a phospholipid. In a more preferred embodiment, the dietary DHA is present in the sn-2 position of the phospholipid carrier of the present invention.

Once hydrolyzed from its esterified molecular phospholipid carrier, DHA may be handled identically by normal metabolic processes. Pancreatic lipase hydrolyses triacylglycerides in the sn-1 and sn-3 positions consistent with the survival of the predominantly saturated sn-2 fatty acid of breast milk post-absorption, whereas intestinal phospholipases in the rat hydrolyze the sn-2 position leaving the predominantly saturated sn-1 position intact. Positional specificity of intestinal glycerolipid hydrolysis ensures that the post-absorptive reassembled phospholipid will have a non-randomized distribution of fatty acids though scrambling among hydrolyzed fatty acids occurs. Positional specificity may be metabolically relevant for subsequent metabolism as fatty acids are incorporated into membranes, oxidized, or excreted on the skin. After reassembly to triglycerides, phospholipids and cholesterol- and fat-soluble vitamin esters in the enterocytes, the absorbed lipids form lipoprotein particles, allowing their transport in the blood for distribution to the various tissues.

In a preferred embodiment, the amount of DHA shows an about 2-fold increase, preferably a 1.9 fold increase in the brain of the young mammal. Additionally or alternatively, the amount of DPA shows a 2- to 4-fold increase, preferably a 2- to 3-fold, more preferably a 2.8 fold increase, in the brain of the young mammal.

In a preferred embodiment, the amount of DPA shows at least a 2-fold increase, more preferably a 2- to 4-fold increase, preferably a 2- to 3-fold, more preferably a 2.8 fold increase, in the brain of the young mammal when using PL-bound DHA compared to (corresponding amounts of) TAG-bound DHA. In a preferred embodiment, the amount of DPA shows at least a 2-fold increase, more preferably a 2- to 4-fold increase, preferably a 2- to 3-fold, more preferably a 2.6 fold increase, in the liver of the young mammal when using PL-bound DHA compared to (corresponding amounts of) TAG-bound DHA. In a preferred embodiment, the amount of DPA shows at least a 2-fold increase, more preferably a 2- to 4-fold increase, preferably a 2- to 3-fold, more preferably a 2.4 fold increase, in the retina of the young mammal when using PL-bound DHA compared to (corresponding amounts of) TAG-bound DHA. In a preferred embodiment, the amount of DPA shows at least a 2-fold increase, more preferably a 2-to 4-fold increase, preferably a 2- to 3-fold, more preferably a 2.8 fold increase, in the synaptosomes of the young mammal when using PL-bound DHA compared to (corresponding amounts of) TAG-bound DHA.

In a preferred embodiment, the increase in the amount of DPA is achieved by a conversion from dietary DHA. More preferably, the increase in the amount of DPA occurs simultaneously and/or concomitantly with and/or as a result of the DHA increase.

In a prefered embodiment, the dietary DHA comprising phospholipid is present in an omega-6 PUFA adequate composition, comprising omega-3 and omega-6 polyunsaturated fatty acids, preferably in a weight ratio of omega-6:omega-3 PUFAs of at least 2:1.

The composition comprising dietary DHA comprising phospholipid preferably comprises linoleic acid (LA) and alpha-linolenic acid (ALA), preferably in a weight ratio of LA:ALA of at least 2:1, more preferably between 6:1 and 16:1.

The composition comprising dietary DHA comprising phospholipid preferably comprises at least 0.1 wt%, preferably at least 0.2 wt%, more preferably at least 0.25 wt%, even more preferably at least 0.50 wt% omega-3 PUFAs, based on total fat. The omega-3 content preferably does not exceed 10 wt%, more preferably does not exceed 5 wt% of total fat. In a preferred embodiment at least 50 %, more preferably at least 75 %, most preferably at least 90 % of all DHA in the composition is provided as PL-DHA.

In a preferred embodiment, the dietary DHA comprising phospholipid is present in an infant formula. In one embodiment, the infant formula is supplemented with DHA comprising phospholipid. Preferably, the human infant has an age of between 0 and 36 months, preferably between 0 and 24 months, more preferably between 0 and 12 months. Consequently, in a preferred embodiment, the dietary DHA comprising phospholipid is comprised by an infant formula which is for feeding infants of age of between 0 and 36 months, preferably between 0 and 24 months, more preferably between 0 and 12 months or even 0 to 6 months. In another preferred embodiment, the infant formula is specifically designed for and/or suitable for preterm infants. In one embodiment, the infant is in need of increased DPA levels in tissue and/or organs as described here above. In one embodiment, the infant exhibits physiologically acceptable DHA levels in these tissues and/or organs. In one embodiment, the infant is at risk or suffers from impaired DPA levels in tissue and/or organs as described here above.

In a preferred embodiment, the dietary DHA comprising phospholipid is present in an infant formula comprising an energy level of 60-70 kcal/100ml, further comprising suitable amounts of proteins, fats and carbohydrates.

Preferably, the infant nutrition comprises per 100kcal nutrients the following macro- and micronutrients: 1.8 to 3.0 g protein, total fat content preferably lies between 4.4 and 6.0 g, of which linoleic acid is preferably at least 300mg or lies between 300 and 1200 mg, and of which alpha-linolenic acid is preferably at least 50 mg, preferably the ratio linoleic/ alpha-linolenic acid lies between 5:1 and 15:1, preferably the sum of lauric acid and myristic acid of total fat content lies between 10 and 30 wt%, preferably around 20 wt%, preferably the level of *trans* fatty acids lies around 2-4 wt% of total fat content and erucic acid is preferable present in an amount of around 0.5 to 2 wt% or about 1 wt% of total fat content of the infant formula.

The weight ratio of whey protein versus the total sum of remaining proteins in the infant formula is preferably equal to 60:40 or higher than 0.6. Whey protein may be present in intact or hydrolyzed form.

In a preferred embodiment, the infant formula has a carbohydrate content of between 9 and 14 g per 100 kcal nutrients of which preferably lactose is present in an amount of at least 4.5 g per liter ready to feed formula or alternatively over 85 wt% of total carbohydrates.

In a preferred embodiment, the infant formula according to the present invention comprises phospholipids in an amount of at most 2 g/l ready to feed formula or at most 300 mg/100kcal.

It is further disclosed that the dietary DHA provides for comprising phospholipid for improving the health or sustaining growth and development of a young mammal, preferably a human infant, through increasing the amounts of DPA or both DHA and DPA in tissues and/or organs of said young mammal.

It is further disclosed that the dietary DHA comprising phospholipid may be used as a food supplement for increasing the amount of DPA or DHA and DPA in tissues and/or organs of a young mammal, preferably a human infant.

### Definitions

The term "egg phospholipid" herein relates to phospholipids that are naturally present in eggs, egg yolks in particular. These phospholipids are readily isolated from eggs or egg yolks using a mild ethanol-based extraction method and commercially available in the market for use in infant nutrition. Preferred phospholipids that are of egg origin include phosphatidylcholine, phosphatidylserine, phosphatidylethanolamine, lysophosphatidylcholine, sphingomyelin, lysophosphatidylethanolamine and phosphatidylinositol. Egg phospholipid preferably refers to a phospholipid mixture comprising significant amounts of phosphaticylcholine (PC).

The term "DHA", short for docosahexaenoic acid, herein relates to the dietary long-chain polyunsaturated omega-3 fatty acid LC-PUFA with a carbon chain of 22 atoms and 6 double bounds present therein, its shorthand name is 22:6(n-3) in the nomenclature of fatty acids.

The term "DPA", short for docosapentaenoic acid, herein relates to the dietary long-chain polyunsaturated omega-3 fatty acid LC-PUFA with a carbon chain of 22 atoms and 5 double bounds present therein, its shorthand name is 22:5(n-3) in the nomenclature of fatty acids.

The term "human infant" herein relates to a human having an age of between 0 and 36 months, preferably between 0 and 24 months, more preferably between 0 and 12 months, most preferably between 0 and months. In one embodiment, the human infant is preferably a preterm infant.

### Examples

### Chemical

¹³C-DHA was prepared by us using published methods (Le, P. M., et al. 2007. Biosynthetic production of universally (13)C-labelled polyunsaturated fatty acids as reference materials for natural health product research. Anal Bioanal Chem 389: 241-249) and esterified into lipid classes by Avanti Polar Lipids (Alabaster, AL,US). The TAG tracer (TAG-¹³C-DHA) had ¹³C-DHA in the sn-2 position with unlabeled 16:0 in the sn-1 and sn-3 positions. The tracer representative for PL was phosphatidylcholine (PC) with ¹³C-DHA in the sn-2 position and unlabeled 16:0 in the sn-1 position (PC-¹³C-DHA). Solvents for tissue lipid extraction were HPLC grade from Sigma-Aldrich (St. Louis, MO, US) or Burdick & Jackson (Muskegon, MI, US).

### Animals and diet

The overall study and all procedures involving live piglets were approved by the Institutional Animal Care and Use Committee (IACUC) at Cornell University. Sows (Yorkshire and Landrace cross) were bred with Hampshire boars. After birth, piglets were left to nurse with the sow for at least 48 hours. Twenty piglets, 2 to 4 days of age, were chosen for the study based on the following criteria: weight (about 2.0 kg), gender (10 males and 10 females), health (active and apparently normal, and not the smallest animal in the litter) and sow. Piglets were given a unique number/color combination identifier and then were transported to Large Animal Research and Teaching Unit (LARTU), Cornell University.

At the study site, piglets were housed in individual stainless steel cages and were placed on a single fully balanced commercially available young farm animal milk replacer formula immediately upon arrival.

The fatty acid profile of the formula is presented in Table 1. DHA is present (0.02%w/w) and slightly higher but still low level in PL (0.11% w/w). TAG, PL, and related DHA measurements are presented in Table 2, when appropriate tabulated for a 500 mL feeding used with the dose. As with human milk and human infant formula, total energy from fat was about 47% and of this total, overwhelmingly from TAG (94%). Despite the low DHA concentration in both TAG and PL, the predominant mass of TAG over PL delivered 73% of DHA compared to 27% from PL, again in accord with human milk and other mammalian milks.

**Table 1. Fatty acid profile of piglet milk formula (replacer), as %, w/w.**

| | Sat. FA | Branched/CLA | Monoene FA | 18:2 n-6 | 20:2 n-6 | 20:3 n-6 | 20:4 n-6 | 22:4 n-6 | 18:3 n-3 | 20:3 n-3 | 22:5 n-3 | 22:6 n-3 |
|---|---|---|---|---|---|---|---|---|---|---|---|---|
| TAG | 42.3 | 0.67 | 41.14 | 14.12 | 0.57 | 0.10 | 0.25 | 0.10 | 0.53 | 0.12 | 0.04 | 0.02 |
| PL | 55.8 | 2.90 | 28.71 | 7.35 | 0.34 | 0.95 | 2.63 | 0.12 | 0.69 | 0.00 | 0.38 | 0.11 |

| | | | | | | | | | | | | |
|---|---|---|---|---|---|---|---|---|---|---|---|---|
| *indicative of ruminant fat. FA=Fatty acid. | | | | | | | | | | | | |

**Table 2. Lipid and DHA distribution in piglet milk replacer.**

| | TAG | PL |
|---|---|---|
| g FA per 500 mL | 23.9 | 1.6 |
| Appr. Energy from FA (%) | 43 | 4 |
| % FA | 94 | 6 |
| mg DHA per 500 mL | 4.8 | 1.8 |
| % DHA | 73 | 27 |

Fresh formula was provided every 6 hours for the first week and 8 hours thereafter in individual troughs. All piglets had free access to fresh water. Formula consumption for each group was recorded daily for the first week to insure they thrive, and every third day thereafter. Piglet body weight was collected and recorded every other day in the first week and every third day thereafter. Enrichment, including bedding, contact with other piglets, positive human contact and rubber toys were all provided. The fatty acid composition of milk replacer (piglet milk formula) is shown in Table 1.

### Dose and sampling

On day 16 of life, 16 robust piglets were assigned to two dosing groups balanced with respect to gender, weight, and age but otherwise distributed randomly. Group PC were orally dosed with PC-¹³C-DHA; Group TG were dosed with TG-¹³C-DHA. The four smallest piglets were used as natural abundance isotopic controls and did not receive a dose but were otherwise treated identically to the other groups.

Doses were prepared by dissolving CHCl₃ solutions of PC-¹³C-DHA or TAG-¹³C-DHA in about 1 mL of refined olive oil and driving off the CHCl₃ by gentle heating under flowing dry N₂ over 4 hours. The resulting olive oils were carefully placed on the surface of a few mL of reconstituted piglet formula and sonicated until homogeneous. The resulting labeled formula was split into 1 to 2 mL aliquots gravimetrically and administered orally to piglets, followed by several washes of the vials containing the labeled formulas, at the beginning of a feed. Care was taken to insure all of the labeled formula and the washes, consisting of a total of up to 10 mL, were swallowed by the piglets. Piglets were then provided with their normal meal of 500 mL milk replacer which they routinely consume completely. Doses to piglets corresponded to about 20 mg ¹³C-DHA delivered in PC and 86 mg of ¹³C-DHA delivered in TAG.

Six days after the dose, and on a single day after 20 days of formula, all piglets were euthanized by exsanguination under anesthesia. Blood for fatty acid analysis was collected in tubes with EDTA and spun to prepare red blood cells. The surface few mm of the grey matter on the cerebral cortex superior surface were rapidly collected at necropsy. A piece was immediately used for preparation of synaptosomes, and the remaining grey matter was flash frozen for fatty acid analysis. Retina, heart, liver, biceps femoris muscle and kidney were also harvested, and all tissues were flash frozen and stored at -80°C until prepared for analysis.

Synaptosomes were prepared using Syn-PER Synaptic Protein Extraction Reagent (Thermo Scientific, Waltham, MA, US) according to manufacturer's instructions. Briefly, 1 ml of Syn-PER Reagent was added to ∼100 mg brain tissue samples. Samples were homogenized and homogenate centrifuged at 1,200 g for 10 mins. The supernatant was collected and further centrifuged at 15,000 g for 20 mins. Synaptosomes were recovered as the pellet and lipids were immediately extracted.

### Lipid extraction and analysis

Total lipids were extracted from samples of brain grey matter and white matter, the synaptosome pellet, liver, heart, and whole retina were simultaneously digested and FAME prepared using a one-step method as described in detail previously (Zhou, Y., et al 2008. The influence of maternal early to mid-gestation nutrient restriction on long chain polyunsaturated fatty acids in fetal sheep. Lipids 43: 525-531). For plasma and erythrocytes, the Bligh and Dyer method was employed to extract total lipids and FAME were prepared using 14% BF3 in methanol (Bligh, E., and W. Dyer. 1959. A rapid method of total lipid extraction and purification. Can J Biochem Physiol 37: 911-917). A known quantity of freshly prepared heptadecanoic acid in chloroform (99% pure, Sigma Chemical) was added as an internal standard to tissue samples just before extraction. FAME were dissolved in heptane and stored at -20°C until analysis.

FAME were analyzed using a Hewlett Packard 5890 series II GC-FID with a BPX 70 column (60 m 0.32 mm inner diameter 0.25 µm film; Hewlett Packard, Palo Alto, CA, U.S.A.) and H2 as carrier gas. Quantitative profiles were calculated using the internal standard and an equal weight FAME mixture to derive response factors for each fatty acid.

### ¹³C DHA analysis

Tracer analysis for ¹³C-DHA is performed on FAME mixtures using similar GC column conditions as for quantitative analysis, as has been described in detail previously (Goodman, K. J., and J. T. Brenna. 1992. High sensitivity tracer detection using high-precision gas chromatography-combustion isotope ratio mass spectrometry and highly enriched [U-13C]-labeled precursors. Anal Chem 64: 1088-1095). Instrumentation for tracer analysis is an Agilent 6890 gas chromatograph coupled to a combustion furnace interface, and to a Thermo Scientific 253 isotope ratio mass spectrometer (IRMS). FAME eluting from the GC are combusted to CO2, dried, and admitted to the IRMS. Data processing is as described previously (Wijendran, V., et al. 2002. Efficacy of dietary arachidonic acid provided as triglyceride or phospholipid as substrates for brain arachidonic acid accretion in baboon neonates. Pediatr Res 51: 265-272). Isotope ratios in the conventional high precision notation, δ¹³C, defined previously (Goodman, K. J., and J. T. Brenna. 1992), is converted to fraction of 13201 C. For each fatty acid, the mean isotope ratio of the control group was subtracted from the isotope ratio of the means for the enriched groups to yield an atom fraction enrichment, which was subsequently converted to %Dose which reflects the appearance of tracer in the specific pool. The primary outcome is a relative comparison of the %Dose appearing in the brain grey matter for TAG and PL, respectively. Total %Dose found in liver and retina was calculated directly from their respective weights. The total labeled DHA was estimated in cerebral grey matter and RBC. The relative amount of grey matter in the brain was taken as 60% estimated from human imaging data (Miller, A. K., R. L. Alston, and J. A. Corsellis. 1980. Variation with age in the volumes of grey and white matter in the cerebral hemispheres of man: measurements with an image analyser. Neuropathol Appl Neurobiol 6: 119-132). For RBC, the blood volume was estimated as 8.5% of body weight and hematocrit was about 35%. For grey matter synaptosomes, it was not attempted to estimate the total amount and normalized the %Dose to the highest value found. In all cases, estimated masses apply to both experimental groups and cancel in the primary and secondary outcome calculations and thus do not affect the final results.

### Statistics

Primary Outcome, Relative DHA %Doses from PC and TAG. The primary outcome is the relative %Dose of ¹³C-DHA found in the grey matter of the cerebral cortex in the ¹³C-DHA vs the TAG-¹³C-DHA dosed groups. The %Dose in the two dosing groups were tested for equivalence by one way analysis of variance with p<0.05 considered significant.

### Secondary outcomes

Total unlabeled fatty acids in the various pools were compared by in a pairwise manner in the two dosing groups and were not significantly different, and were therefore pooled. Since these two groups were fed the same formulas and treated identically except for a few mg doses, no differences were expected based on treatment.

Relative ¹³C-DHA %Doses for synaptosomes, retina and liver were compared for similarity to the primary outcome. Relative meal-wise amounts of total DHA delivered in TAG and PL were calculated from the determination of relative amounts of cold DHA in formula in a 500 mL meal.

### Results

Piglets have long been used as an accepted model for infant nutrition because of its similar metabolism to humans and in numerous studies have been used to investigate the aspects of LC-PUFA delivery from TAG vs. PL. Moreover, the pig is generally considered a good model for human brain development because it is among the few experimental animals with a brain growth spurt similar to humans, and it is a large non-ruminant omnivore.

Piglets in both dosing groups grew at the same rates and attained non-significantly different final weights of 9.4±0.3 kg and 9.1±0.4 kg for the phosphatidylcholine (PC) and triacylglyceride (TAG) dosed groups, respectively. The isotopic control groups were purposely chosen as the smallest animals and grew in parallel to the other groups, starting at about 6% lower body weight and finishing at about 12% lower weight. These animals are expected to yield accurate estimates of baseline isotope ratios, which are applied identically to both experimental groups.

¹³C label was detected for DHA and 22:5n-3 (DPAn-3) only among all fatty acids. The highest %Dose was detected in cerebral cortex grey matter in the ¹³C-DHA-PC dosed animals, at about 0.4%. ¹³C-DHA from the TAG dose was about half this value, thus dietary PC was 1.9-fold more efficacious for supplying DHA to the developing piglet brain than TAG. Results in grey matter synaptosomes were consistent with the relative values. The ratio of relative accretion of labeled DHA was 1.7-fold greater for PC than for TAG.

The converted DPA n-3 product was labeled below 0.05%Dose for the PC dose and less than half that for the TAG dose. Surprisingly, the relative efficacy of PC over TAG was significantly different with a ratio of about 2.8 for cerebral cortex grey matter and for grey matter synaptosomes, the superiority of PC over TAG was the same 2.8-fold.

Liver retained 6.8% of the PC ¹³C-dose compared to 3.5% of the TAG dose at 6 days post-dose. The relative efficacy was 1.9, similar to the grey matter and synaptosomes. The liver ¹³C-DHA was about 20-fold greater overall than the grey matter. Surprisingly, DPAn-3 was significantly increased with a 2.6-fold higher amount in the liver for PC compared to TAG. In grey matter, ¹³C-DHA was about 8-fold greater than ¹3C-DPA while in liver the 13C-DHA was 40-fold greater.

For the retina, relative efficacy of PC over TAG is 2.2-fold for DHA, and surprisingly, significantly different at about 2.4-fold for DPAn-3, all similar and consistent with results in other tissues examined. Consistent with the brain but in contrast to the liver, ¹³C-DHA is 6.3-fold greater than DPAn-3, again suggesting that conversion took place outside the liver and possibly in the retina. Again, TAG predominates as the source of retina DHA.

The data presented herein show conversion of DHA to ¹³C-22:5n-3, and a parallel advantage for the use of PL-bound DHA. No labeling was found in 20:5n-3 which would have been expected if 18:3n-3 was elongated with labeled acetate, and no evidence of label in other fatty acids, suggesting that the acetate pool was not a factor in the conversion from DHA to DPA. These considerations all suggest that the label in 22:5n-3 did not proceed via acetate.

## Claims

1. A composition for use in increasing the amount of DPA or the amount of DHA and DPA in tissues and/or organs of a human infant at risk of or suffering from impaired DPA levels in tissue and/or organs, wherein said composition comprises a DHA comprising phospholipid (PL-DHA) and comprises at least 0.1 wt% omega-3 PUFAs, based on total fat.

2. A composition for use in increasing the amount of DPA or the amount of DHA and DPA in tissues and/or organs of a human infant suffering from impaired DPA levels in tissue and/or organs, wherein said composition comprises a DHA comprising phospholipid (PL-DHA).

3. The composition for use according to claim 1 or 2, wherein the amount of DPA or the amount of DHA and DPA is/are increased in the brain, eyes and liver of the human infant.

4. The composition for use according to any one of the preceding claims, wherein the tissues and/or organs comprise or consist of neural tissue, brain tissue, cerebral cortex grey matter, brain grey matter, synaptosomes, retina or the liver of the human infant.

5. The composition for use according to any one of the preceding claims, wherein the phospholipid is selected from the group consisting of phosphatidylcholine, phosphatidylserine, phosphatidylethanolamine, lysophosphatidylcholine, sphingomyelin, lysophosphatidylethanolamine and phosphatidylinositol, preferably phosphatidylcholine or a mixture of egg phospholipids comprising phosphatidylcholine in an amount of between 60 and 80 wt% of the phospholipid mixture.

6. The composition for use according to any one of the preceding claims, wherein the dietary DHA is present in the sn-2 position of the phospholipid carrier.

7. The composition for use according to any one of the preceding claims, wherein the dietary DHA comprising phospholipid (PL-DHA) is present in an infant formula.

8. The composition for use according to any one of the preceding claims, wherein the human infant has an age of between 0 and 36 months, preferably between 0 and 24 months, more preferably between 0 and 12 months.

9. The composition for use according to any one of the preceding claims, wherein the increase in the amount of DPA is achieved by a conversion from the dietary DHA.

10. The composition for use according to any one of the preceding claims, wherein the increase in the amount of DPA occurs simultaneously and/or concomitantly with and/or as a result of the DHA increase.

11. A non-therapeutic method for supporting healthy brain development of a young mammal, said method involving increasing the amount of DPA or the amount of DHA and DPA in tissues and/or organs of a young mammal, by allowing the young mammal to consume a dietary DHA comprising phospholipid (PL-DHA), wherein the composition comprises at least 0.1 wt% omega-3 PUFAs, based on total fat, said young mammal preferably being a human infant.

## Patentansprüche

1. Zusammensetzung für die Verwendung bei der Erhöhung der Menge an DPA oder der Menge an DHA und DPA in Geweben und/oder Organen eines menschlichen Säuglings/Kleinkinds mit dem Risiko von, oder leidend an, beeinträchtigten DPA-Spiegeln in Gewebe und/oder Organen, wobei die Zusammensetzung ein DHA umfasst, das Phospholipid (PL-DHA) umfasst, und wenigstens 0,1 Gewichts-% Omega-3-PUFAs basierend auf dem Gesamtfett umfasst.

2. Zusammensetzung für die Verwendung bei der Erhöhung der Menge an DPA oder der Menge an DHA und DPA in Geweben und/oder Organen eines menschlichen Säuglings/Kleinkinds, das an beeinträchtigten DPA-Spiegeln in Gewebe und/oder Organen leidet, wobei die Zusammensetzung ein DHA umfasst, das Phospholipid (PL-DHA) umfasst.

3. Zusammensetzung für die Verwendung nach Anspruch 1 oder 2, wobei die Menge an DPA oder die Menge an DHA und DPA im Gehirn, den Augen und der Leber des menschlichen Säuglings/Kleinkinds erhöht ist/sind.

4. Zusammensetzung für die Verwendung nach einem der vorhergehenden Ansprüche, wobei die Gewebe und/oder Organe umfassen oder bestehen aus Nervengewebe, Gehirngewebe, graue Substanz des Großhirns, graue Substanz des Gehirns, Synaptosome, Retina oder der Leber des menschlichen Säuglings/Kleinkinds.

5. Zusammensetzung für die Verwendung nach einem der vorhergehenden Ansprüche, wobei das Phospholipid ausgewählt ist aus der Gruppe bestehend aus Phosphatidylcholin, Phosphatidylserin, Phosphatidylethanolamin, Lysophosphatidylcholin, Sphingomyelin, Lysophosphatidylethanolamin und Phosphatidylinositol, bevorzugt Phosphatidylcholin oder eine Mischung von Ei-Phospholipiden, die Phosphatidylcholin in einer Menge zwischen 60 und 80 Gew.-% der Phospholipidmischung umfassen.

6. Zusammensetzung für die Verwendung nach einem der vorhergehenden Ansprüche, wobei das diätische DHA in der sn-2-Position des Phospholipidträgers vorhanden ist.

7. Zusammensetzung für die Verwendung nach einem der vorhergehenden Ansprüche, wobei das diätische DHA, das Phospholipid (PL-DHA) umfasst, in einer Säuglingsanfangs-/Kleinkindnahrung vorhanden ist.

8. Zusammensetzung für die Verwendung nach einem der vorhergehenden Ansprüche, wobei der/das menschliche Säugling/Kleinkind ein Alter von 0 bis 36 Monaten, bevorzugt zwischen 0 und 24 Monaten, bevorzugter zwischen 0 und 12 Monaten hat.

9. Zusammensetzung für die Verwendung nach einem der vorhergehenden Ansprüche, wobei der Anstieg in der Menge an DPA durch Umwandlung aus dem diätischen DHA erzielt wird.

10. Zusammensetzung für die Verwendung nach einem der vorhergehenden Ansprüche, wobei der Anstieg in der Menge an DPA gleichzeitig und/oder begleitend mit und/oder als ein Ergebnis des DHA-Anstiegs auftritt.

11. Nichttherapeutisches Verfahren für die Unterstützung der gesunden Gehirnentwicklung eines jungen Säugetiers, wobei das Verfahren das Steigern der Menge an DPA oder der Menge an DHA und DPA in Geweben und/oder Organen eines jungen Säugetiers beinhaltet, durch Ermöglichen, dass das junge Säugetier ein diätisches DHA konsumiert, das das Phospholipid (PL-DHA) umfasst, wobei die Zusammensetzung wenigstens 0,1 Gewichts-% Omega-3-PUFAs basierend auf dem Gesamtfett umfasst, wobei das junge Säugetier bevorzugt ein menschlicher/s Säugling/Kleinkind ist.

## Revendications

1. Composition à utiliser pour augmenter la quantité de DPA ou la quantité de DHA et de DPA dans des tissus et/ou des organes d'un nourrisson humain à risque ou souffrant de niveaux de DPA altérés dans des tissus et/ou organes, dans laquelle ladite composition comprend un phospholipide comprenant du DHA (PL-DHA) et comprend au moins 0,1 % en poids de PUFA oméga-3, sur la base de la graisse totale.

2. Composition destinée à être utilisée pour augmenter la quantité de DPA ou la quantité de DHA et de DPA dans des tissus et/ou des organes d'un nourrisson humain souffrant de niveaux de DPA altérés dans des tissus et/ou des organes, dans laquelle ladite composition comprend un phospholipide du DHA (PL-DHA).

3. Composition à utiliser selon la revendication 1 ou 2, dans laquelle la quantité de DPA ou la quantité de DHA et de DPA est/sont augmentées dans le cerveau, les yeux et le foie du nourrisson humain.

4. Composition à utiliser selon l'une quelconque des revendications précédentes, dans laquelle les tissus et/ou organes comprennent ou sont constitués de tissu neural, de tissu cérébral, de matière grise du cortex cérébral, de matière grise cérébrale, de synaptosomes, de rétine ou du foie du nourrisson humain.

5. Composition à utiliser selon l'une quelconque des revendications précédentes, dans laquelle le phospholipide est choisi dans le groupe constitué de la phosphatidylcholine, de la phosphatidylsérine, de la phosphatidyléthanolamine, de la lysophosphatidylcholine, de la sphingomyéline, de la lysophosphatidyléthanolamine et du phosphatidylinositol, de préférence de la phosphatidylcholine d'un mélange de phospholipides d'œuf comprenant de la phosphatidylpholiphine en une quantité comprise entre 60 et 80 % en poids du mélange de phospholipides.

6. Composition à utiliser selon l'une quelconque des revendications précédentes, dans laquelle le DHA alimentaire est présent en position sn-2 du support phospholipidique.

7. Composition à utiliser selon l'une quelconque des revendications précédentes, dans laquelle le phospholipide comprenant du DHA alimentaire (PL-DHA) est présent dans une préparation pour nourrisson.

8. Composition à utiliser selon l'une quelconque des revendications précédentes, dans laquelle le nourrisson humain a un âge compris entre 0 et 36 mois, de préférence entre 0 et 24 mois, de manière plus préférée entre 0 et 12 mois.

9. Composition à utiliser selon l'une quelconque des revendications précédentes, dans laquelle l'augmentation de la quantité de DPA est obtenue par une conversion à partir de DHA alimentaire.

10. Composition à utiliser selon l'une quelconque des revendications précédentes, dans laquelle l'augmentation de la quantité de DPA survient simultanément et/ou concomitamment avec et/ou en conséquence de l'augmentation de DHA.

11. Méthode non thérapeutique pour soutenir le développement sain du cerveau d'un jeune mammifère, ladite méthode impliquant l'augmentation de la quantité de DPA ou de la quantité de DHA et de DPA dans des tissus et/ou des organes d'un jeune mammifère, en permettant au jeune mammifère de consommer un phospholipide contenant du DHA alimentaire (PL-DHA), dans laquelle la composition comprend au moins 0,1% en poids de PUFA oméga-3, sur la base de la graisse totale, ledit jeune mammifère étant de préférence un nourrisson humain.
